Europäisches Patentamt

European Patent Office

Office européen des brevets

(19)

(11) Veröffentlichungsnummer: **0 265 548**
**B1**

(12) ## EUROPÄISCHE PATENTSCHRIFT

(45) Veröffentlichungstag der Patentschrift:
12.09.90

(51) Int. Cl.⁵: **A61K 35/14**

(21) Anmeldenummer: 86115097.7

(22) Anmeldetag: 30.10.86

(54) Verfahren zur Herstellung von keimabgetöteten oder in ihrer Virulenz abgeschwächten Substanzen sowie deren Verwendung.

(43) Veröffentlichungstag der Anmeldung:
04.05.88 Patentblatt 88/18

(45) Bekanntmachung des Hinweises auf die Patenterteilung:
12.09.90 Patentblatt 90/37

(84) Benannte Vertragsstaaten:
AT BE CH DE ES FR GB GR IT LI LU NL SE

(56) Entgegenhaltungen:
EP-A- 29 893
EP-A- 153 995
DE-A- 3 109 691

K.E.Theurer "Innovative Biotherapie", Hippokrates
Verlag, Stuttgart 1987
Tierärztliche Umschau 37(8), Aug.1982, Seite 580-583
Therapie Woche 35(13) März 1985, Seite 154
"Der Kassenarzt" 21(12) 1981, Seite 3-8

(73) Patentinhaber: Kief, Horst, Dr. med., Londoner Ring 105,
D-6700 Ludwigshafen(DE)

(72) Erfinder: Kief, Horst, Dr. med., Londoner Ring 105,
D-6700 Ludwigshafen(DE)

ACTORUM AG

## Beschreibung

Die Erfindung betrifft ein Verfahren nach der im Anspruch 1 definierten Gattung sowie die Verwendung der durch dieses Verfahren hergestellten Substanz, kurz "Suspension" genannt, insbesondere zur Beeinflussung des Immunsystems im menschlichen und/oder tierischen Organismus.

Nach dem derzeitigen Stand der Wissenschaft und Technik in der Medizin gibt es verschiedene Möglichkeiten, das Immunsystem im Körper zu beeinflussen:

Etwa durch passive oder aktive Impfung, d.h. durch Stimulation von Antikörpern oder durch direkte Zufuhr von Antikörpern, wobei auf das immunmodulatorische Geschehen entweder suppressiv (unterdrückend) oder stimulativ eingewirkt werden kann (Definitionen medizinischer Fachausdrücke insbesondere nach "Pschyrembel, Klinisches Wörterbuch", Walter de Gruyter, Berlin – New York, 1972).

Eine besondere Bedeutung hat die sogenannte Desensibilisierung gewonnen, bei der das auslösende Antigen zunächst in sehr starker Verdünnung und dann in steigender Dosierung dem Organismus zugeführt wird, um das überschießende Antiköpergeschehen zu neutralisieren. Nachteile dieses Verfahrens sind die geringe Erfolgsquote, die sehr lang dauernde Behandlungszeit und das relativ eng umschränkte Indikationsgebiet allergischer Erkrankungen.

Ein einfaches und dabei erfolgreiches Verfahren ist die Injektion von Eigenblut. Hierbei wurde ein Verfahren bekannt, bei welchem Eigenblutverdünnungen mit Aluminiumhydroxidsuspensionen versetzt werden, analog der Impfstoffbindung an Aluminiumhydroxid bei bislang bekannten Verfahren, wobei durch die Proteinbindung an das Oxid sicherlich nicht nur ein gewisser Depoteffekt für das Erythrozytenmaterial und die Plasmaeiweißkörper erzielt wird, sondern auch eine teilweise Verfremdung immunrelevanter Eiweißkörper:

Auch dieses Verfahren ist nicht zu einer größeren Bedeutung gelangt, da ihm eine gesicherte Wirksamkeit in breiterem Rahmen verzagt blieb.

Erwähnt werden soll auch eine Außenseitermethode, bei der Patientenblut durch ein Ozon-Sauerstoff-Gemisch sehr stark oxidiert und danach dem Organismus zurückgegeben wird. Einzelbeobachtungen sprechen dafür, daß auch hier immunmodulatorische Vorgänge ausgelöst werden.

Mach dem in der EP-B 0 153 995 (Theurer) beschriebenen Verfahren wird eine Säuredampflyse von biologischen Wirkstoffen angegeben, die der schonenden Sterilisierung durch Abtötung von Mikroorganismen und Viren, etwa über Persäuren, dienen soll. Daher können auch Peroxyde entstehen, die dann eine Reizwirkung ähnlich wie Ozon zur Folge haben, aber wegen ihrer zu scharfen Wirkung toxisch wirkende Produkte entstehen lassen. Sie sind einer Oxydation als nur ähnlich anzusehen und das Verfahren hat negative Nebeneffekte zur Folge. Daher wird in dieser Literaturstelle auch ein gewolltes Rückgängig-Machen der "Schein-Oxydatio" vorgeschlagen.

Die Verwendung von Suspensionen zur Oxydationstherapie wird insbesondere auch in der DE-PS 3 109 691 (Kief) beschrieben, wo auf die Eigenblutteprapie nach Wolff hingewiesen wird, noch mehr auf die hyperbare Ozontherapie unter Verwendung von Venenblut nach Wehrli, für welche dort eine Vorrichtung zur extrakorporalen Keimabtötung angegeben wird. Auch das Oxydationsverfahren nach Wehrlie wird angeführt.

Bei den Vorschlägen nach Wolff und Kief wird also körpereigenes Blut – als Ursprungssubstanz – und nur dieses einem Oxydationsvorgang unterworfen und danach als solches reinfundiert; dieses geschieht so in ausschließlicher Weise; auch erfolgt nur ein Oxydationsvorgang.

Der Erfindung liegt die Beobachtung zugrunde, daß oxydiertes Eigenblut – in vereinzelten Fällen – sowohl suppressiv als auch in anderen, ähnlich gelagerten Fällen stimulativ wirken kann. Aufklärung in diese widersprüchliche Beobachtung brachte die Erkenntnis, daß oxydiertes bzw. ozonisiertes Plasma häufig immunsuppressiv wirkt, während ebenso behandeltes Erythrozytenkonzentrat ebenso häufig immunstimulativ wirkt. Aber auch gegenläufige Prozesse können ausgelöst werden. Eine weitere Aufschlüsselung des Geschehens führte zu der Erkenntnis, daß selbst deproteinisiertes Serum noch immunmodulatorische Vorgänge auslösen kann.

Auch Urin, in diesem Zusammenhang etwas vereinfacht als durch die Niere vom Blut abfiltrierte und selectiv konzentrierte Elektrolytlösung zu betrachten, kann, als Injektion verabreicht, Immunprozesse auslösen.

Der Einsatz von Urin als "Desensibilisierungsmittel" wurde früher in der naturheilkundlichen Medizin zwar oft geübt, ist aber zwischenzeitlich in Vergessenheit geraten, da die bislang üblichen desinfizierenden Zusätze, Thymol und Phenol, selbst teilweise toxisch und nicht als Zusätze geeignet sind. Den Urin andererseits unfiltriert und unsterilisiert zu verwenden verbietet sich aber auf Grund der Gefahr von Spritzenabszessen.

Ausgehend von diesen Erkenntnissen wird bei dem eingangs definierten Verfahren erfindungsgemäß vorgeschlagen, die Ursprungssubstanzen zu fraktionieren, wobei diese nicht nur menschliches oder tierisches Blut sein können sondern auch ein Gewebe oder eine über Kulturen vermehrte Ursprungssubstanz, aber auch Urin.

Das Verfahren nach der Erfindung wird nachstehend beschrieben, wobei noch Weiterbildungen der Erfindung angegeben werden:

Die Ursprungssubstanz, etwa das Blut des erkrankten Individuums, wird in bekannter Weise in eine sterile Plasmaflasche aufgesaugt und heparinisiert, um es ungerinnbar zu halten. Die Menge des zuzugebenden Heparins richtet sich naturgemäß nach der Menge des aufgesaugten Blutes. Danach wird restliche Luft aus der Flasche abgesaugt und dann in bekannter Weise, etwa nach der in der eingangs erwähnten Patentschrift (Kief) beschriebenen, ein Ozon-Sauerstoff-Gemisch unter Druck eingeblasen. Statt des Blutes als Ausgangssubstanz kann also wie angegeben Gewebe und/oder Urin Verwendung finden. Letzterer erfüllt dann ebenfalls alle Anforderungen an ein untoxisches, gut verträgliches und vor allem wirksames Immunmodulans.

Diese Ursprungssubstanz wird also gem. dem tragenden Gedanken der Erfindung fraktioniert. Die Fraktionierung selbst kann dabei vor der, gfls. erstmaligen, Oxydation erfolgen, aber auch erst danach oder, bei mehrfacher Oxydation, dazwischen. Das Fraktionieren wird also in den Ablauf des geschilderten bekannten Verfahrens eingeschoben.

In den nachfolgenden Erläuterungen zur Erfindung werden auch noch gleichzeitig die möglichen Ursachen für die überraschende Wirkung der Erfindung dargelegt:

1. Die zellulären Bestandteile des menschlichen und tierischen Blutes, weißes und rotes Blutbild und Thrombozyten, liegen eingebettet in die Proteine des Plasmas, diese wiederum gelöst in Serum, das Ganze also ein equilibriertes und gepuffertes System, das sich nach den Prinzipien der Regulation und Gegenregulation selbst schützt und erhält. Wird nun das Blut fraktioniert, dann entfallen die gegenseitigen regulativen Schutzmechanismen der einzelnen Systeme und die selectierte Fraktion ist, etwa am Beispiel gewaschener Erythrozyten, ohne den schützenden Proteinmantel einer Noxe unmittelbar ausgesetzt, sodaß dann eine bewußt gesteuerte, teilweise Verfremdung der Proteine von zellulären Bestandteilen des Blutes einen wesentlich stärkeren immunmodulatorischen Effekt ausüben kann.

2. Der Zusatz von Ozon oder sonstigen (starken) Oxydantien zur immunmodulatorischen Verfremdung von Blut oder Blutfraktionen darf als sehr naturnaher Prozeß bezeichnet werden, da der Körper selbst im Rahmen der körpereigenen Abwehr häufig auf oxidative Prozesse zurückgreift, etwa die weißen Blutkörperchen bei der Infektabwehr im Rahmen des "respiratory burst" mittels Sauerstoffradikalen, darunter der mittels Oxydation ablaufende Teil der körpereigenen Abwehr zu verstehen ist.

3. Die meisten Erreger, Bakterien oder Viren, weisen eine organotrope Wirkung auf, d.h. sie besiedeln, bezogen auf unser Organ "Blut", lediglich bestimmte Bestandteile desselben, beispielsweise das Aids-Virus eine bestimmte Lymphozytensubpopulation, sodaß die viruzide, fungizide und bakterizide Wirkung des Oxydans erst nach Fraktionierung des Blutes zu einer optimalen spezifischen Antigen-Vernichtung und somit gezielten Immunstimulation führt.

4. Die Zelle kann sich vor dem starken oxidativen Angriff durch besondere Fermentausstattung in der Zellmembran schützen. Erst nach Aufbrechen und/oder Zerlegen der Struktur sind sehr viele wichtige immunmodulatorische Substanzen der Oxidation zugänglich. Dieses Aufbrechen und Zerlegen schützender Strukturen kann geschehen durch mechanischen Einfluß (Homogenisierung), unphysiologische Temperatur (Einfrieren), Osmose oder eiweißspaltende Fermente, etwa Pepsin, Papain oder Bromelain. Die dabei zutagetretenden "Bruchstellen" bestimmter Eiweißfraktionen mit neuen potentiellen Angriffsstellen für Ozon, Sauerstoff und/oder die ionisierende Strahlung unterstreichen die Bedeutung einer mehrfachen Oxydation des Blutes bzw. selectierter Blutfraktionen.

Die hier in Zusammenhang mit "Blut" getroffenen Aussagen gelten, im Rahmen der Erfindung und vereinfacht ausgedrückt, letztlich auch für die anderen erwähnten Ursprungssubstanzen.

Bei der Oxydation ist es von untergeordneter Bedeutung, ob das Oxydans dem jeweiligen Medium als Fertigprodukt aus der Siemens'schen Entladungsröhre zugeführt wird oder ob es beispielsweise im zu behandelnden, Material selbst durch Aufsättigung mit Sauerstoff und gfls nochmaliges Bestrahlen mit UV-Licht generiert wird.

Beachtet man die Organotropie verschiedener Antigene und der dadurch in den verschiedenen Systemen gebundenen Antikörper, dann wird der nächste Schritt einer gezielten Immunstimulation bei einer evtl. ebenfalls gewünschten selectiven Immunsuppressiom in einem anderen Organsystem deutlich:

Dazu wird vorgeschlagen, die fraktionierte Ursprungssubstanz nach gem. der Erfindung getrennter Behandlung der Fraktionen danach wieder teilweise oder insgesamt zu einer einheitlichen Suspension zusammenzufügen. Dabei kann es sinnvoll sein, die einzelnen Fraktionen vorher über eigene Kulturen zu vermehren, beispielsweise eine Lymphozytenpopulation als Träger bestimmter Antikörper. Man erzeugt demnach durch die selective Oxydation einer derart vermehrten "Blutfraktion" eine konzentrierte Antikörpersuspension, die durch das Oxydans in "physiologischer Weise" verfremdet, bei erneutem Kontakt mit dem Organismus einen spezifischen Anti-Autoantikörperprozeß auslöst, der den ursprünglich pathologischen Immunisationsvorgang kompensiert. Der erneute Kontakt mit dem Spenderorganismus kann dabei parenteral hergestellt werden, d.h. durch Injektion, oder oral, d.h. durch Einnehmen, aber auch durch Inhalation oder durch reinen Hautkontakt, etwa durch Einreiben.

Dargestellt am Beispiel Blut erhält man also durch nochmaliges Zusammenfügen von selectierten und getrennt einer Oxydation unterworfenen, insbesondere ozonisierten Fraktionen oder Hämolysaten der weißen und roten Blutzellreihen, des Plasmas, des defibrinierten Serums und/oder des Urins zu einer einheitlichen Dilution ein Medikament, das sowohl über die gezielten immunstimulativen Eigenschaften eines Aktivimpfstoffes einerseits verfügt, andererseits gleichzeitig aber auch, sofern therapeutisch sinnvoll, eine immunsuppressive, organbezogene Wirkung, ähnlich einem Passivimpfstoff. Unter Hämolysat ist im Sinne der Erfindung eine Suspension aus Plasma und Intrazellularflüssigkeit der Erythrozyten von wechselnder Zusammensetzung zu verstehen.

Praktisch geht man dabei so vor, daß aus einem, wie eingangs beschrieben, mit einem Oxydans behandeltem Eigenblut die Erythrozyten abzentrifugiert werden, das Plasma abgezogen, nochmals getrennt mit einem Oxydans behandelt, die Erythrozyten nach (mehrmaligem) Waschen erneut mit Oxydans behandelt, teilweise in Aqua destillata suspendiert und so auf osmotischem Wege zum Platzen gebracht und nochmals mit einem Oxydans behandelt werden. Bei besonders gelagerten Fällen wird entweder dem Serum oder der Erythrozytensuspension mit einem Oxydans behandeltes Urinfiltrat zugefügt. Die gewünschte Hämolyserate läßt sich dabei in idealer Weise durch eine variable Sauerstoff-/ Ozon- Konzentration steuern. Es wurde gefunden, daß die Konzentration des Ozon-Sauerstoff-Gemisches im Idealfall zwischen 40 und 80 ng $O_3$ pro ml $O_2$ liegt.

Man erhält nach Rekombination der genannten Fraktionen, eventuell auch mit dem Urinfiltrat, ein äußerst potentes Immunmodulans, das je nach mengenmäßiger Zusammensetzung sowohl über immunstimulative als auch über immunsuppressive Eigenschaften verfügt. Verfährt man auf die geschilderte Art und Weise, beispielsweise mit dem Blutserum einer bestimmten Patientengruppe, etwa Polyarthritikern, dann erhält man ein Poolserum, das erfolgreich gegen Rheuma einzusetzen ist.

Durch Zusatz von Carbonylgruppenträgern aromatischer oder aliphatischer Struktur bei der Zugabe des Oxydans kann deren scharfe oxydatische Potenz in physiologische Bahnen gelenkt werden, ähnlich den Chinonen im Atmungsprozeß. Es wird daher in Weiterbidlung der Erfindung vorgeschlagen, je nach der gewünschten Schärfe des Oxidationsprozesses an einem bestimmten Punkt der OxydansZugabe etwa Ascorbat (Vitamin C) zu zufügen, das durch seine Umwandlung in Dehydroascorbinsäure während des chemischen Prozesses als idealer Carbonylgruppenträger aromatischer Struktur anzusehen ist. Die Umwandlung des Vitamin C in Dehydroascorbinsäure kann dabei an jedem beliebigen Punkt des Prozesses durch Gefrieren gestoppt werden, besonders durch Schockgefrieren. Auch Carbonylgruppenträger aliphatischer Struktur können Verwendung finden.

In besonders gelagerten Fällen kann die immunstimulative Wirkung der rekombinierten Suspension so stark sein, daß sie besonders in der Anfangsphase der Behandlung nur verdünnt, etwa 1:10, zum Einsatz kommen sollte. Diese Verdünnung ist mit ozonisiertem Aqua injectabile (spritzfähiges Wasser) besonders vorteilhaft; es ist nicht nur absolut keimfrei sondern auch einer länger dauernden Lagerung der verdünnten Suspension förderlich.

## Patentansprüche

1. Verfahren zur Herstellung von keimabgetöteten oder in ihrer Virulenz abgeschwächten Substanzen (Suspensionen) auf extrakorporalen Wege, wobei die aus einem lebenden, erkrankten Individuum entnommenen Ursprungssubstanzen einer Oxydation mittels Ozon, Sauerstoff und/oder ionisierender Strahlung unterworfen werden, dadurch gekennzeichnet, daß die Ursprungssubstanzen fraktioniert werden.

2. Verfahren nach Anspruch 1, dadurch gekennzeichnet, daß die Ursprungssubstanz Gewebe ist.

3. Verfahren nach Anspruch 1, dadurch gekennzeichnet, daß die Ursprungssubstanz über Kulturen vermehrt wird.

4. Verfahren nach Anspruch 1, dadurch gekennzeichnet, daß die Ursprungssubstanz Urin ist.

5. Verfahren nach einem oder mehreren der vorhergehenden Ansprüche, dadurch gekennzeichnet, daß die Ursprungssubstanz auf Unterzellgröße zerkleinert wird (Zellyse).

6. Verfahren nach Anspruch 5, dadurch gekennzeichnet, daß die Zellyse auf mechanischem, enzymatischem und/oder osmotischem Wege durchgeführt wird.

7. Verfahren nach einem oder mehreren der vorhergehenden Ansprüche, gekennzeichnet durch wiederholte Oxydation.

8. Verfahren nach einem oder mehreren der vorhergehenden Ansprüche, dadurch gekennzeichnet, daß die Fraktionen jeweils für sich filtriert und erneut einer oder erst dann der Oxydation unterworfen werden.

9. Verfahren nach einem oder mehreren der vorhergehenden Ansprüche, gekennzeichnet durch eine bei der Oxydation erfolgende Zugabe von Carbonylgruppenträgern.

10. Verfahren nach Anspruch 9, dadurch gekennzeichnet, daß die Carbonylgruppenträger aromatischer Struktur sind.

11. Verfahren nach Anspruch 9, dadurch gekennzeichnet, daß die Carbonylgruppenträger aliphatischer Struktur sind.

12. Verfahren nach einem oder mehreren der vorhergehenden Ansprüche, dadurch gekennzeichnet, daß der Ozongehalt des $O_2$–$O_3$-Gemisches gesteuert wird.

13. Verfahren nach einem oder mehreren der vorhergehenden Ansprüche, dadurch gekennzeichnet, daß die Konzentration des $O_2$–$O_3$-Gemisches 40 bis 80 ng $O_3$ pro ml $O_2$ beträgt.

14. Verfahren nach einem oder mehreren der vorhergehenden Ansprüche, dadurch gekennzeichnet, daß bei der Oxydation zusätzlich Ascorbinsäure zugesetzt wird.

15. Verfahren nach einem oder mehreren der vorhergehenden Ansprüche, dadurch gekennzeichnet, daß die Ursprungssubstanz oder deren Fraktionen nach der Oxydation verdünnt werden.

16. Verfahren nach Anspruch 15, dadurch gekennzeichnet, daß die Verdünnung mittels ozonisiertem Wasser erfolgt.

17. Verfahren nach einem oder mehreren der vorhergehenden Ansprüche, dadurch gekennzeichnet, daß behandelte Fraktionen wieder zu einer einheitlichen Dilution zusammengefügt werden.

18. Verfahren nach einem oder mehreren der vorhergehenden Ansprüche, dadurch gekennzeichnet, daß die fertige Suspension schockgefroren wird.

19. Verfahren nach einem oder mehreren der vorhergehenden Ansprüche, dadurch gekennzeichnet, daß die fertige Suspension gefriergetrocknet wird.

## Claims

1. Process for the preparation of substances (suspensions) which have been sterilized or whose virulence has been attenuated, by extra corporeal means, where the original substances which have been removed from a living, diseased individual are subjected to an oxidation by means of ozone, oxygen and/or ionising radiation, characterized in that the original substances are fractionated.

2. Process according to Claim 1, characterized in that the original substance is tissue.

3. Process according to Claim 1, characterized in that the original substance is multiplied via cultures.

4. Process according to Claim 1, characterized in that the original substance is urine.

5. Process according to one or more of the preceding claims, characterized in that the original substance is reduced to subcellular size (celllysis).

6. Process according to Claim 5, characterized in that the cell lysis is carried out by mechanical, enzymatic and/or osmotic means.

7. Process according to one or more of the preceding claims, characterized by repeated oxidation.

8. Process according to one or more of the preceding claims, characterized in that the fractions are each filtered separately and subjected to a renewed oxidation or only then subjected to the oxidation.

9. Process according to one or more of the preceding claims, characterized by addition of carbonyl-group carriers taking place at the oxidation.

10. Process according to Claim 9, characterized in that the carbonyl-group carriers are of aromatic structure.

11. Process according to Claim 9, characterized in that the carbonyl-group carriers are of aliphatic structure.

12. Process according to one or more of the preceding claims, characterized in that the ozone content of the $O_2$-$O_3$ mixture is controlled.

13. Process according to one or more of the preceding claims, characterized in that the concentration of the $O_2$-$O_3$ mixture is 40 to 80 ng of $O_3$ per ml of $O_2$.

14. Process according to one or more of the preceding claims, characterized in that ascorbic acid is additionally added at the oxidation.

15. Process according to one or more of the preceding claims, characterized in that the original substance or fractions thereof are diluted after the oxidation.

16. Process according to Claim 15, characterized in that the dilution takes place by means of ozonised water.

17. Process according to one or more of the preceding claims, characterized in that treated fractions are combined again to a uniform dilution.

18. Process according to one or more of the preceding claims, characterized in that the finished suspension is shock-frozen.

19. Process according to one or more of the preceding claims, characterized in that the finished suspension is freeze-dried.

**Revendications**

1. Procédé pour la production par une voie extra-corporelle de substances (suspensions) à germes tués ou dont la virulence est atténuée, les substances de départ, provenant d'un individu vivant atteint d'une maladie, étant soumises à une oxydation au moyen d'ozone, d'oxygène et/ou d'un rayonnement ionisant, caractérisé en ce que les substances de départ sont fractionnées.

2. Procédé selon la revendication 1, caractérisé en ce que la substance de départ est un tissu.

3. Procédé selon la revendication 1, caractérisé en ce que la substance de départ est multipliée par culture.

4. Procédé selon la revendication 1, caractérisé en ce que la substance de départ est l'urine.

5. Procédé selon une ou plusieurs des revendications précédentes, caractérisé en ce que la substance de départ est fragmentée jusqu'à une taille infracellulaire (lyse cellulaire).

6. Procédé selon la revendication 5, caractérisé en ce que la lyse cellulaire est effectuée par un moyen mécanique, enzymatique et/ou osmotique.

7. Procédé selon une ou plusieurs des revendications précédentes, caractérisé par une oxydation répétée.

8. Procédé selon une ou plusieurs des revendications précédentes, caractérisé en ce que les fractions sont chacune pour soi filtrées et soumises à nouveau une oxydation ou seulement alors à l'oxydation.

9. Procédé selon une ou plusieurs des revendications précédentes, caractérisé par une addition de porteurs de groupes carbonyle, effectuée lors de l'oxydation.

10. Procédé selon la revendication 9, caractérisé en ce que les porteurs de groupes carbonyle ont une structure aromatique.

11. Procédé selon la revendication 9, caractérisé en ce que les porteurs de groupes carbonyle ont une structure aliphatique.

12. Procédé selon une ou plusieurs des revendications précédentes, caractérisé en ce que l'on règle la teneur en ozone du mélange $O_2$-$O_3$.

13. Procédé selon une ou plusieurs des revendications précédentes, caractérisé en ce que la concentration du mélange $O_2$-$O_3$ est de 40 à 80 ng d'$O_3$ par ml d'$O_2$.

14. Procédé selon une ou plusieurs des revendications précédentes, caractérisé en ce que l'on ajoute en outre de l'acide ascorbique lors de l'oxydation.

15. Procédé selon une ou plusieurs des revendications précédentes, caractérisé en ce que, après l'oxydation, on dilue la substance de départ ou des fractions de celle-ci.

16. Procédé selon la revendication 15, caractérisé en ce que la dilution est effectuée à l'aide d'eau ozonisée.

17. Procédé selon une ou plusieurs des revendications précédentes, caractérisé en ce que les fractions traitées sont à nouveau réunies en une dilution unique.

18. Procédé selon une ou plusieurs des revendications précéaentes, caractérisé en ce que la suspension finale est soumise à une brusque congélation.

19. Procédé selon une ou plusieurs des revendications précédentes, caractérisé en ce que la suspension finale est lyophilisée.